# EUROPEAN PATENT SPECIFICATION

(11) **EP 0 783 693 B1**
(45) Date of publication and mention of the grant of the patent: **07.11.2001**
(21) Application number: 95931864.3
(22) Date of filing: 22.09.1995
(51) Int. Cl.: G01N 33/50, C12N 15/12, C12N 5/06

(54) **IN VITRO MODELS OF CNS FUNCTION AND DYSFUNCTION**
IN VITRO-MODELLE FÜR CNS-FUNKTION UND-DYSFUNKTION
MODELES IN VITRO DE FONCTION ET DE DYSFONCTION DU SYSTEME NERVEUX CENTRAL

(30) Priority: 23.09.1994 US 311099; 07.06.1995 US 481893
(43) Date of publication of application: 16.07.1997
(60) Divisional application: 01101622.7
(73) Proprietor: NEUROSPHERES HOLDINGS LTD., Calgary, Alberta T2L 2KY (CA)
(72) Inventor: WEISS, Samuel, Calgary, Alberta T2L 1A6 (CA); REYNOLDS, Brent, Salt Spring Island,BC V8K 1X9 (CA)
(74) Representative: Barth, Renate, Dr.
(86) International application number: CA9500542
(87) International publication number: WO9609543

(56) References cited:
- EP-A- 0 233 838
- WO-A-91/09936
- WO-A-93/01275

## Description

### Background of the Invention

The mature human nervous system is composed of billions of cells that are generated during development from a small number of precursors located in the neural tube. The study of central nervous system (CNS) developmental pathways as well as alterations that occur in adult mammalian CNS due to dysfunction have been difficult due to the complexity of the mammalian CNS. Such matters would be better studied using relatively simple models of the CNS under defined conditions.

Generally, two approaches have been taken for studying cultured CNS cells: the use of primary neural cultures; and the use of neural cell lines. Primary mammalian neural cultures can be generated from nearly all brain regions providing that the starting material is obtained from fetal or early post-natal animals. In general, three types of cultures can be produced, enriched either in neurons, astrocytes, or oligodendrocytes. Primary CNS cultures have proven valuable for discovering many mechanisms of neural function and are used for studying the effects of exogenous agents on developing and mature cells. While primary CNS cultures have many advantages, they suffer from two primary drawbacks. First, due to the limited proliferative ability of primary neural cells, new cultures must be generated from several different animals. While great care is usually taken to obtain tissue at identical states of development and from identical brain regions, it is virtually impossible to generate primary cultures that are identical. Hence, there exists a significant degree of variability from culture to culture.

A second disadvantage of primary cultures is that the tissue must be obtained from fetuses or early post-natal animals. If primary cultures are to be performed on a regular basis, this requires the availability of a large source of starting material. While this is generally not a problem for generating primary cultures from some species (e.g. rodents), it is for others (e.g. primates). Due to the limited supply and ethical concerns, the culturing of primary cells from primates (both human and non-human) is not practical.

Due to the limited proliferative ability of primary neural cells, the generation of a large number of homogenous cells for studies of neural function, dysfunction, and drug design/screening has previously not been achieved. Therefore, homogenous populations of cells that can generate a large number of progeny for the in vitro investigation of CNS function has been studied by the use of cell lines. The generation of neural cell lines can be divided into two categories: 1) spontaneously occurring tumors, and 2) custom-designed cell lines.

Of the spontaneously occurring tumors, probably the most studied cell line for neurobiology is the rat pheochromocytoma (PC12) cells that can differentiate into sympathetic-like neurons in response to nerve growth factor (NGF). These cells have proven to be a useful model for studying mechanisms of neural development and alterations (molecular and cellular) in response to growth factors. Neuroblastoma and glioma cell lines have been used to study neuronal and glial functioning (Lies, et al., 1987; Nister et al., 1988). Embryonal carcinoma (EC) cells are derived from teratoma tumors of fetal germ cells and have the ability to differentiate into a large number of non-neural cell types with some lines (e.g. P19 cells, Jones-Villeneuve et al., 1982) having the ability to differentiate into neural cells (McBurney et al., 1988). A human teratocarcinoma-derived cell line, NTera 2/cl.D1, with a phenotype resembling CNS neuronal precursor cells, can be induced to differentiate in the presence of retinoic acid. However, the differentiated cells are restricted to a neuronal phenotype [Pleasure and Lee (1993), J. Neurosci. Res. 35: 585-602]. While these types of cell lines are able to generate a large number of cells for screening the effects of exogenous agents on cell survival or function, due to their immortalization, they are not suitable for use in the study of apoptosis, i.e. natural programmed death of mammalian cells. In addition, the limited number of these types of lines, the limited number of phenotypes that they are able to generate and the unknown nature of their immortalization (which may effect the function of the cells in an undefined manner) makes these types of cell lines less than ideal for in vitro models of neural function and discovery of novel therapeutics.

An alternative approach to spontaneously occurring cell lines is the intentional immortalization of a primary cell by introducing an oncogene that alters the genetic make-up of the cell thereby inducing the cell to proliferate indefinitely. This approach has been used by many groups to generate a number of interesting neural cell lines (Bartlett et al., 1988; Frederiksen et al., 1988; Trotter et al., 1989; Ryder et al. 1889; Murphy et al., 1991; Almazan and McKay, 1992). While these lines may prove useful for studying the decisions that occur during cell determination and differentiation, and for testing the effects of exogenous agents, they suffer from several drawbacks. First, the addition of an oncogene that alters the proliferative status of a cell may affect other properties of the cell (oncogenes may play other roles in cells besides regulating the cell cycle). This is well illustrated in a study by Almazan and McKay (1992) and their immortalization of an oligodendrocyte precursor from the optic nerve which is unable to differentiate into type II astrocytes (something that normal optic nerve oligodendrocyte precursors can do). The authors suggest the presence of the immortalizing antigen may alter the cells ability to differentiate into astrocytes.

Another drawback to using intentionally immortalized cells results from the fact that the nervous system is composed of billions of cells and possibly thousands of different cell types, each with unique patterns of gene expression and responsiveness to their environment. A custom-designed cell line is the result of the immortalization of a single progenitor cell and its clonal expansion. While a large supply of one neural cell type can be generated, this approach does not take into account cellular interactions between different cell types. In addition, while it is possible to immortalize cells from a given brain region, immortalization of a desired cell is not possible due to the lack of control over which cells will be altered by the oncogene. Hence, while custom designed cell lines offer a few advantages over spontaneously occurring tumors, they suffer from several drawbacks and are less than ideal for understanding CNS function and dysfunction.

WO 93/01275 discloses the use of neural stem cells cultured *in vitro* for the screening of potential neurologically therapeutic compositions. WO 91/09936 discloses methods of proliferating neuron progenitor cells. European patent application 0 233 838 discloses the preparation of cDNA libraries prepared from glial cells.

### Summary of the Invention

In light of the deficiencies attendant with the prior art methods of providing large quantities of genetically unaltered neural cells for the purpose of studying CNS development and function and for use in determining the effects of potential therapeutic agents for CNS dysfunction, there exists a need for improved CNS model systems that can be used for these purposes.

Accordingly, it is an object of the present invention to provide a CNS model system for the studies of neural development and function and for determining the CNS effects of novel therapeutics and other biological agents. It is an object of this invention that such a CNS system allow for the generation of a large number of cells from a relatively small amount of starting material obtained from a variety of species, including humans and extending over a wide age range, including adults.

It is an object to provide a CNS model system that comprises cells that are not spontaneously occurring tumors or have not been intentionally immortalized by the insertion of an oncogene in order to induce unlimited proliferation, thereby removing any questions of the influence of genetic alteration on the normal function of the cells.

It is another object to provide a CNS model system wherein the cells are clonally derived and thus represent a population of cells having a low degree of variability from one use of the model to the next use.

It is another object of the invention to provide a CNS model system wherein the cells proliferate in response to an extrinsic signaling molecule, or combination of molecules, that can be added or removed at will.

Another object is to provide a CNS model system wherein the proliferated cells can be maintained in an undifferentiated state and allowed to differentiate, when desired, into the three major cell types of the mammalian CNS (neurons, astrocytes, and oligodendrocytes).

It is an object of the present invention to provide a CNS model system whereby the differentiation and functioning of CNS cells can be studied in a controlled manner in a system composed of multiple cell types ― a situation similar to what occurs in vivo.

It is a further object to provide a CNS model system whereby CNS stem cells can be generated from pre- and post-natal individuals, including adults, allowing for testing to be done on an individual basis.

It is an object of the present invention to allow for the analysis of gene expression in the CNS stem cells and stem cell-derived progeny of a normal donor and in the cells of a patient with a neurological disorder.

The objects are accomplished by a method as defined in the appending Claims.

### Brief Description of the Drawings

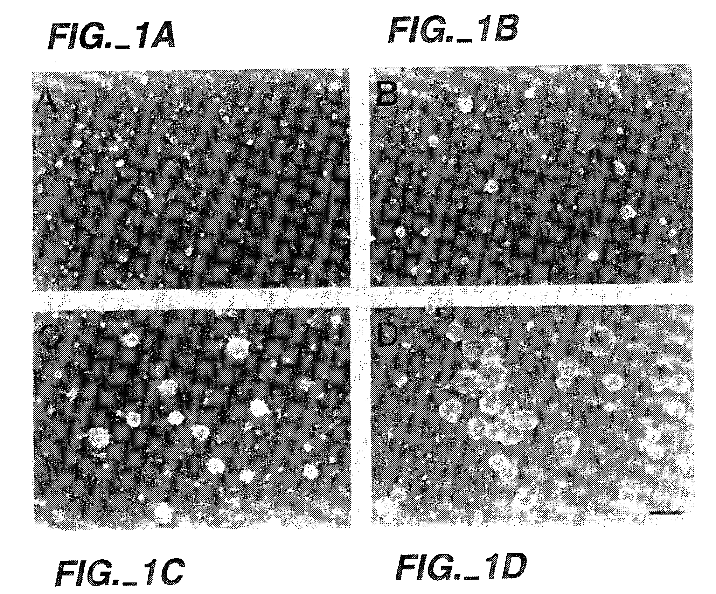

**Fig. 1. Proliferation Of Epidermal Growth Factor (EGF) Responsive Cells:** After 2 days in vitro EGF-responsive cells begin to proliferate (Fig. 1A). After 4 days in vitro small clusters of cells are apparent (Fig. 1B). The clusters of continuously proliferating cells continue to grow in size (Fig. 1C) until they lift off the substrate and float in suspension (Fig. 1D). At this stage, the floating spheres can be easily removed, dissociated into single cells and, in the presence of EGF, proliferation can be re-initiated. (Bar: 50 *µ*m).

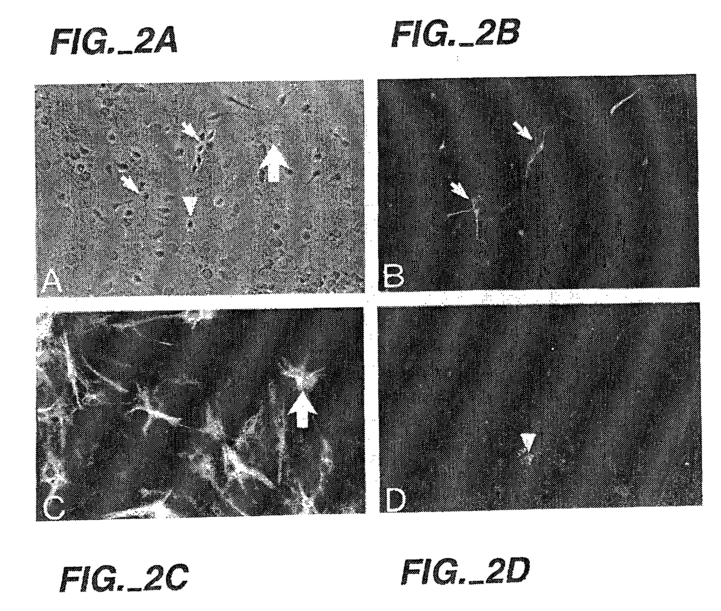

**Fig. 2. Differentiation Of Cells From Single EGF-Generated Spheres Into Neurons, Astrocytes, And Oligodendrocytes:** Triple-label immunocytochemistry with antibodies to microtubule associated protein (MAP-2), glial fibrillary acidic protein (GFAP), and 04 (a cell surface antigen) are used to detect the presence of neurons (Fig. 2B), astrocytes (Fig. 2C) and oligodendrocytes (Fig. 2D), respectively, from a single EGF-generated sphere (Fig. 2A) derived from primary culture. (Bar: 50 *µ*m).

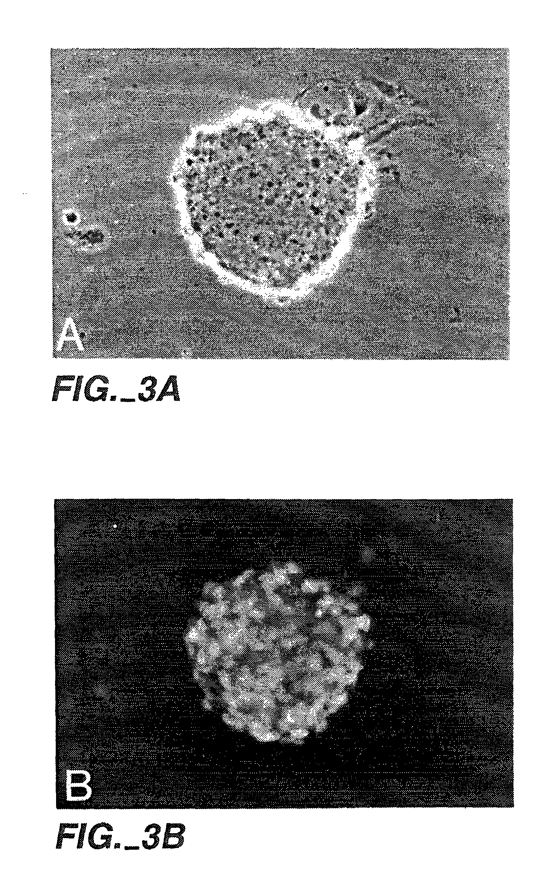

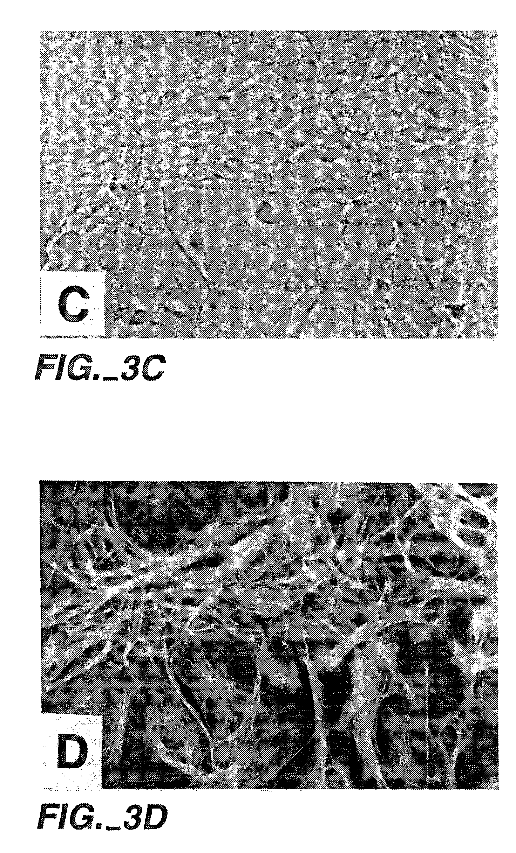

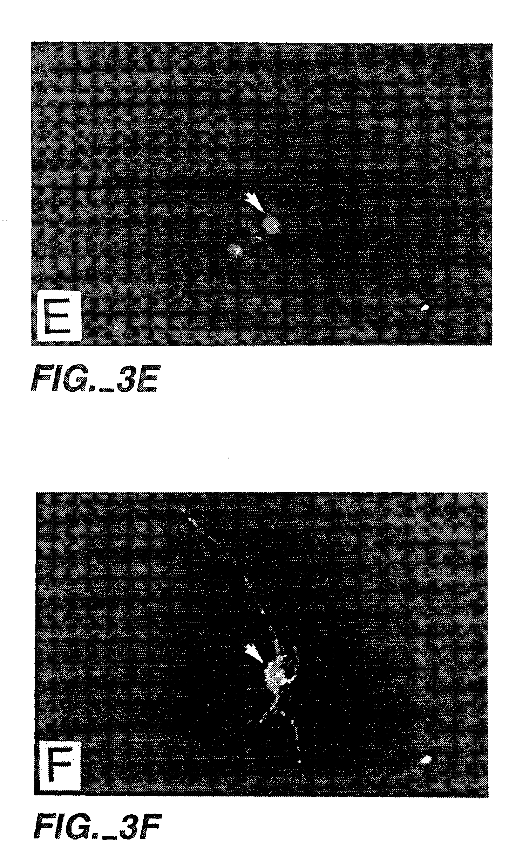

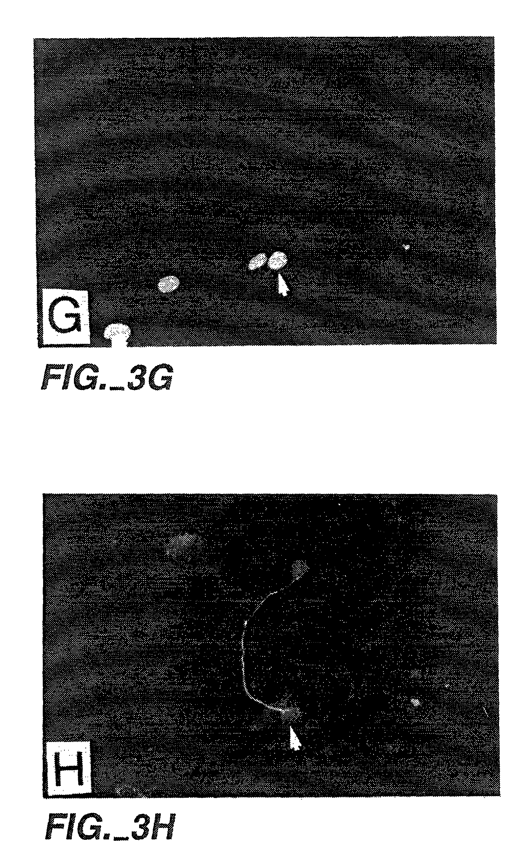

**Fig. 3. Labeling Of Neurospheres Co-Cultured With Striatal Astrocytes:** A phase contrast view of an 8 day old neurosphere grown on an astrocyte feeder layer is shown in Fig 3A. Brd-U labeling of neurosphere cells shows that virtually all cells incorporate Brd-U (Fig. 3B). Phase contrast of the feeder layer cells is shown in Fig. 3C. GFAP labeling of the feeder layer cells shows that the majority of cells in the feeder layer are astrocytes (GFAP-IR) (Fig. 3D). After differentiation occurs, BrdU labeled neurons (Figs. 3E and 3G) are immunoreactive for neuropeptide Y (NPY) (Fig. 3F) or somatostatin (Fig. 3H), as well as other neurotransmitters such as glutamate and methenkephalin (not shown).

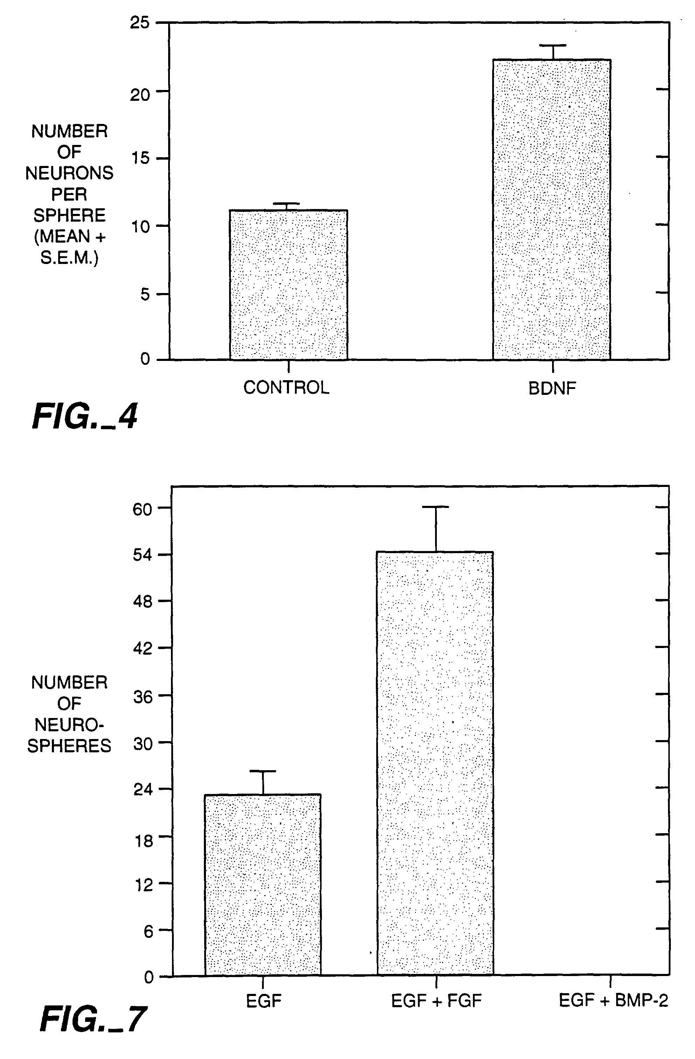

**Fig. 4. Increased Numbers Of Neurons Produced From One Neurosphere In Presence of Brain-Derived Neurotrophic Factor (BDNF):** Quantification of the mean number of neurons at 10 days in vitro (DIV) from single EGF-responsive stem cell-generated neurospheres showed that in the absence of BDNF, 11.46 ± 1.21 neurons per neurosphere were generated. When BDNF (10 ng/ml) was present in the culture medium, a significantly greater (p < 0.5) number of neurons were identified (22.34 ± 2.33 neurons per neurospheres).

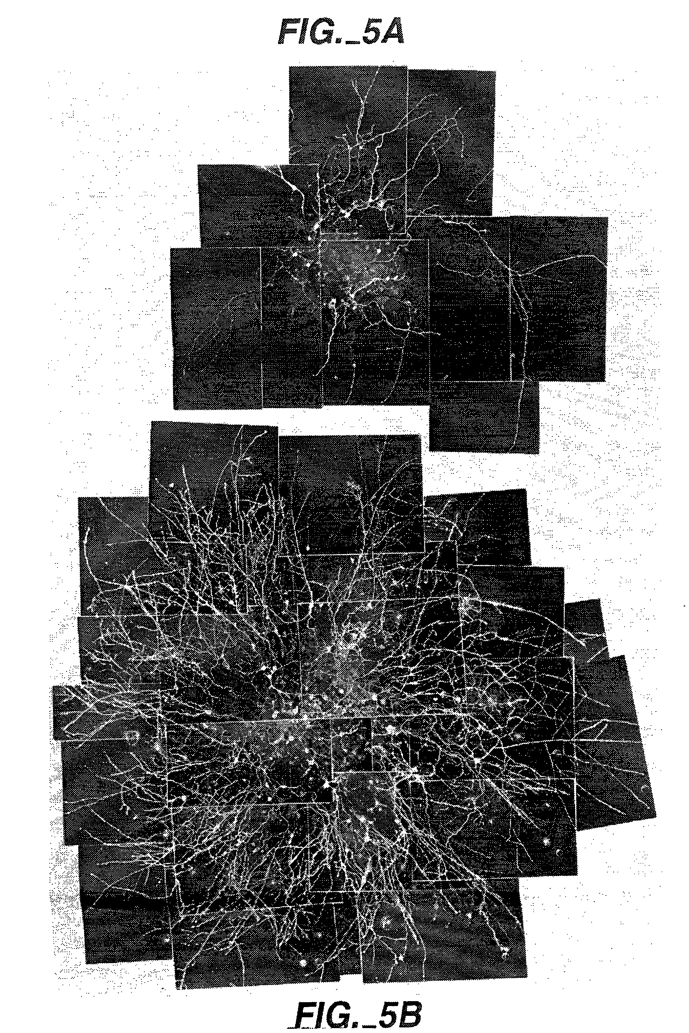

**Fig. 5. Enhanced Neuronal Process Outgrowth In Presence of BDNF:** Neurospheres, grown in the absence of BDNF (Fig. 5A) and in the presence of BDNF (Fig. 5B) for 10 DIV, were fixed and processed for indirect immunocytochemistry with antiserum to _{*T*}-amino butyric acid (GABA). The majority of neurons grown in the presence of BDNF extended long neurites and exhibited an extensive and complex branching pattern relative to the non-BDNF treated neurospheres.

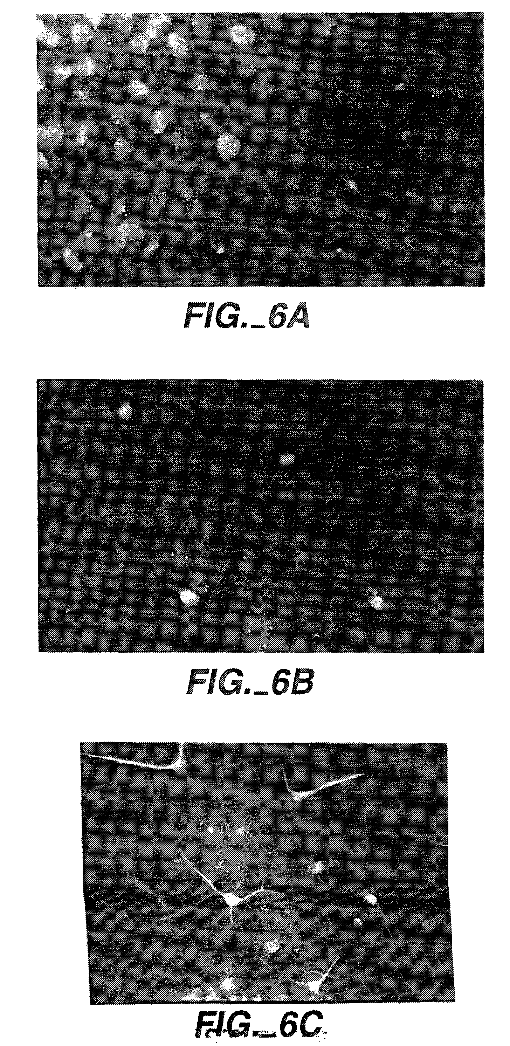

**Fig. 6. Response To BDNF By Selective Populations Of Cells Within A Neurosphere:** Indirect immunocytochemistry for the immediate-early gene product c-fos reveals that nearly all of the cells within a single clonally derived neurosphere are responsive to EGF (20 ng/ml) stimulation, as assayed by increased c-fos immunoreactivity (Fig. 6A). Dual-label immunocytochemistry with antiserum to the nuclear antigen c-fos and an antibody directed against the neuronal specific antigen β-tubulin, demonstrates that a 60 minute exposure to BDNF results in a selective expression of c-fos (Fig. 6B), primarily in the neuronal population as determined with the β-tubulin antisera (Fig. 6C).

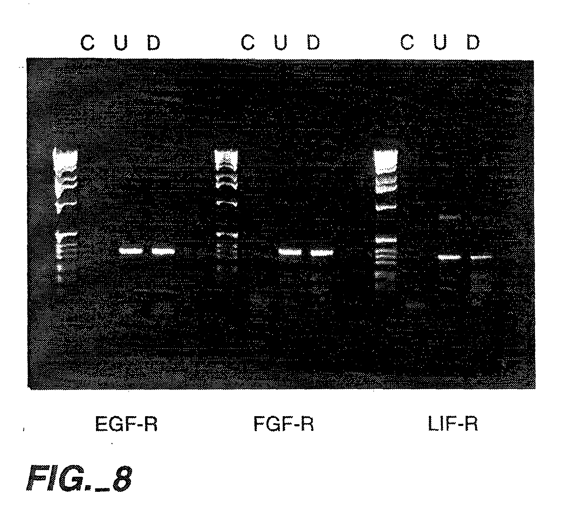

**Fig. 7. Effect Of Basic Fibroblast Growth Factor (bFGF) And Bone Morphogenic Protein 2 (BMP-2) On Proliferation Of EGF-Generated Neurospheres:** Cells isolated from the striatum of the 14 day old embryonic mouse were plated into a 96 well plate at a density of 25,000 cells/ml in the presence of EGF (20 ng/ml. EGF + bFGF (each at 20 ng/ml.) or EGF + BMP-2 (20 and 10 ng/ml respectively). After 10 DIV, quantification of EGF treated cultures indicated that 23 ± 1.33 neurospheres were generated per well (n = 8). bFGF enhanced EGF-stimulated proliferation by giving rise to 54.5 ± 2.17 neurospheres per well (n = 8), while BMP-2 prevented stem cell proliferation in response to EGF (n = 8).
**Fig. 8. Ethidium Agarose Gel Visualized Via UV Transillumination Showing The Detection of Growth Factor Transcripts In Undifferentiated And Differentiated Stem Cell-Derived Progeny:** The first lane in each panel shows a 1 kb standard molecular weight ladder. The second lane, labeled C, is the negative control which represents PCR in the absence of any cDNA template. The third lane, labeled U, is the RT-PCR of undifferentiated neurospheres. The fourth lane, labeled D, is the RT-PCR of differentiated stem cell-derived progeny. The presence of epidermal growth factor receptor, fibroblast growth factor receptor and leukemia inhibitory factor receptor, are indicated by EGF-R, FGF-R and LIF-R, respectively.

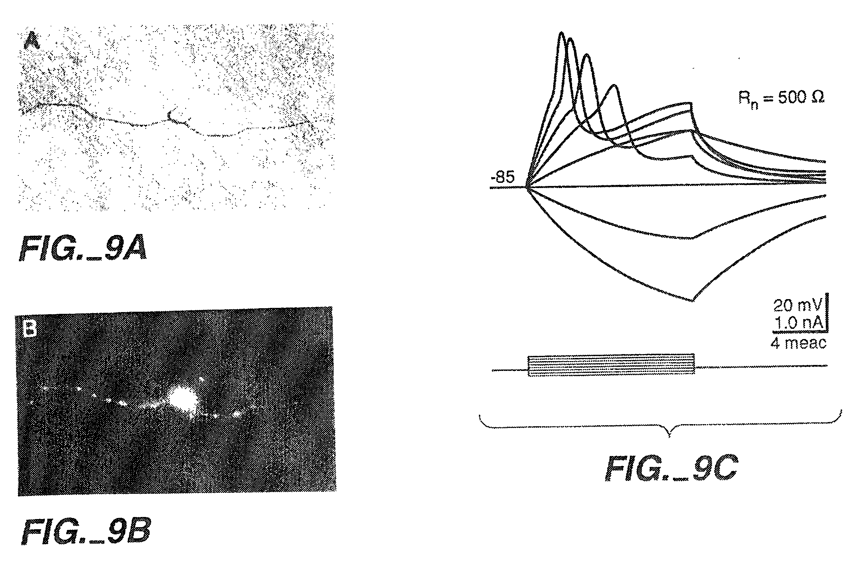

**Fig. 9. Electrophysiological Properties of bFGF-generated Neurons:** A digital image of a presumed neuron illustrating bipolar morphology before patch recording is shown in Fig. 9A. Fig. 9B shows a fluorescence digital image of the same neuron, filled with 5-carboxyfluoroscein, following withdrawal of the patch electrode. Fig. 9C depicts graded action potentials evoked by current injection, in the bFGF-generated neuron illustrated in Figs. 9A and 9B.

### Detailed Description of the Invention

Neural stem cells of the central nervous system (CNS), referred to herein as "CNS stem cells", have been reported and their potential use described (Reynolds and Weiss, Science 255:1707 [1992]; Reynolds et al., *J*. *Neurosci. 12*:4565 [1992]; Reynolds and Weiss, *Restorative Neurology and Neuroscience 4*:208 [1992]; Reynolds and Weiss, *Neuronal Cell Death and Repair,* ed. Cuello [1993]). The term "stem cell" refers to a relatively quiescent undifferentiated cell which can be obtained from embryonic, juvenile, or adult tissue that is capable of proliferation and self-maintenance with the generation of a large number of progeny. Like stem cells found in other mammalian tissues, the CNS stem cell exhibits the critical feature of a stem cell, self-maintenance. Self-maintenance in a cell implies that the cell is able to generate clones of itself and hence maintain its phenotype over an extended period of time.

The stem cell progeny are referred to herein as "precursor cells" and consists of two types of cells: a) new stem cells and b) progenitor cells that can differentiate into functional cells.

The term "progenitor cell" refers to an undifferentiated cell derived from a CNS stem cell. The progenitor cell has limited proliferative ability and cannot self-renew. It is committed to a particular path of differentiation and will, under appropriate conditions, differentiate into the different cell types present in the CNS; these include neurons, and glial cells. Glial cell types include astrocytes and oligodendrocytes.

The term "oligodendrocyte" refers to a differentiated glial cell which forms the myelin surrounding axons in the central nervous system (CNS). Oligodendrocytes are of the phenotype galactocerebroside (+), myelin basic protein (+), and glial fibrillary acidic protein (-) [GalC(+), MBP(+), GFAP(-)]. The term "astrocyte" refers to a differentiated glial cell that is GFAP(+), GalC(-), and MBP(-) which can have a flat protoplasmic/fibroblast-like morphology or which can display a stellate process bearing morphology. The term "neuron" refers to a differentiated neuronal cell having the phenotype neuron specific enolase (+), neurofilament (+), microtubule associated protein (+), Tau-1 (+) or β-tubulin (+) [NSE(+), NF (+), MAP-2 (+), Tau-1 (+), or β-tub (+)]. Accordingly the terms "neural stem cell" or "CNS stem cell", as used herein, refer to multipotent stem cells capable of proliferation to produce more multipotent stem cells and progenitor cells that differentiate into neurons, astrocytes and oligodendrocytes.

Neural stem cells can be isolated and cultured *in vitro* from mammalian CNS by the methods described in Example 1 below. In brief, the stem cells, which have been obtained from mammalian (e.g. human, monkey, rat, mouse, etc.) tissue, are grown in a defined serum-free medium in the presence of at least one growth factor. As used herein, the term "growth factor" refers to a protein, peptide or other molecule having a growth, proliferative, differentiative, or trophic effect on the stem cells and/or progenitor cells. Growth factors which may be used for inducing proliferation include any factor which allows the cells to proliferate, including any molecule which binds to a receptor on the surface of the cell to exert a growth-inducing or survival effect on the cell. Such factors include acidic and basic fibroblast growth factors (aFGF and bFGF which is also known as FGF-2), platelet-derived growth factor (PDGF), thyrotropin releasing hormone (TRH), epidermal growth factor (EGF), an EGF-like ligand, amphiregulin, transforming growth factor alpha (TGFα), brain-derived neurotrophic factor (BDNF), ciliary neurotrophic factor (CNTF), glial-derived neurotrophic factor (GDNF), insulin-like growth factor (IGF-1) and the like. A preferred growth factor is EGF. Also preferred is bFGF or the combination of EGF and bFGF. Growth factors used to regulate stem and progenitor cell development, which have regulatory actions on the cells other than the promotion of proliferation, include transforming growth factor beta (TGFβ), retinoic acid, activin, bone morphogenic protein (BMP), ciliary neurotrophic factor (CNTF) and macrophage inflammatory proteins (MIP-1α, MIP-1β, MIP-2).

In the presence of a growth factor, a multipotent stem cell is induced to divide giving rise to a cluster of undifferentiated cells which is referred to herein as a "neurosphere". The neurosphere is comprised primarily of multipotent stem cells and progenitor cells. Collectively, the cells of the neurosphere are referred to herein as "precursor cells". In vitro, precursor cells typically grow in the form of neurospheres, but they may exhibit different growth patterns depending upon culturing conditions and techniques. Initially, the cells of the neurosphere are not immunoreactive for GFAP, NF, NSE or MBP. However, the cells are of the nestin (+) phenotype, an intermediate filament protein found in undifferentiated CNS cells. The nestin marker was characterized by Lehndahl et al., Cell 60:585-595 (1990). The mature phenotypes associated with the cell types which may be differentiated from the progeny of the cells of the neurosphere are predominantly negative for the nestin phenotype.

In the continued presence of a mitogen such as EGF or the like, precursor cells within the neurosphere continue to divide resulting in an increase in the size of the neurosphere and the number of undifferentiated cells [nestin(+), GFAP(-), NF(-), NSE (-), MBP (-)]. At this stage, the cells are non-adherent and tend to form the free-floating clusters characteristic of neurospheres. After 6 to 7 DIV, the cells of the neurosphere can be dissociated. Virtually all of the cells attach to the tissue culture substrate. In the continued presence of a growth factor, stem cells begin to divide and lift off the substrate forming new free-floating neurospheres consisting of clonally-derived cells. Hence, utilizing this method of proliferation, dissociation, and re-initiation of proliferation, an unlimited number of clonally-derived precursor cells can be generated in vitro.

Upon removal of the mitogenic growth factor, proliferation of the stem cell ceases. The sphere of undifferentiated cells can be adhered to a substrate such as polyornithine-treated plastic or glass where the cells begin to differentiate into neurons and glial cells. Thus, the growth factor acts as an extrinsic signaling molecule that can be added or removed at will to control the extent of proliferation.

When the mitogenic growth factor is removed, the growth-factor responsive stem cell progeny can be co-cultured on a feeder layer. Many types of feeder layers may be used, such as fibroblasts, neurons, astrocytes, oligodendrocytes, tumor cell lines, genetically altered cell lines or any cells or substrate with bioactive properties. The feeder layer generally produces a broader range of phenotypes. In this instance, the feeder layer acts as a substrate and source of both membrane bound and soluble factors that induce and alter the differentiation of the stem cell-generated progeny. Compared to a more inert substance, such as poly-L-ornithine, an astrocyte feeder layer, for example, induces a broader range of neuronal phenotypes as determined by indirect immunocytochemistry at 7 DIV. When differentiated on a poly-L-ornithine coated substrate with 1 % fetal bovine serum, neuronal phenotypes are almost exclusively GABAergic or Substance Pergic. When differentiated on an astrocyte feeder layer, in addition to GABAergic and Substance Pergic neurons, somatostatin, neuropeptide Y (NPY), glutamate and met-enkephalin-containing neurons are present. The astrocytes can be derived from tissue obtained from various brain regions such as the striatum, cortex and spinal cord.

Once the growth factor is removed, the culture medium may contain serum such as 0.5-1.0% fetal bovine serum (FBS). Serum tends to support the differentiation process and enhance cell survival, especially when the differentiating cells are grown at a low density.

Within 1-3 days after removal of the growth factor and placing of the cell in conditions that support differentiation and survival, most or all of the precursor cells begin to lose immunoreactivity for nestin and begin to express antigens specific for neurons, astrocytes or oligodendrocytes. The identification of neurons is confirmed using immunoreactivity for NSE, NF, β-tub, NeuN (a nuclear antigen), MAP-2 and the neuron specific protein Tau-1. Astrocytes and oligodendrocytes are identified using immunoreactivity for GFAP and GalC, respectively. Cells that do not express antigens specific for neurons or for astrocytes, begin to express markers specific for oligodendrocytes in a correct temporal fashion. That is, the cells first become immunoreactive for 04 (a cell surface antigen), GalC (a myelin glycolipid) and finally, MBP. These cells also possess a characteristic oligodendrocyte morphology.

Neurons can also be identified based on their specific neurotransmitter phenotype together with analysis of their morphology. Using single, dual, or triple-label immunofluorescence and immunoperoxidase methods, differentiated neurosphere cultures can be analyzed for the expression of neurotransmitters, or in some cases for the enzymes responsible for the neurotransmitter synthesis. Alternatively, in situ hybridization histochemistry can be performed using cDNA or RNA probes specific for the peptide neurotransmitter or the neurotransmitter synthesizing enzyme mRNAs. These techniques can be combined with immunocytochemical methods to enhance the identification of specific phenotypes. If necessary, the antibodies and molecular probes discussed above can be applied to Western and Northern blot procedures respectively to aid in the cell identification.

In addition to being able to isolate EGF-responsive stem cells from any region in the embryonic CNS, CNS stem cells can also be isolated from a variety of juvenile and adult CNS regions, using routine biopsy procedures, including the conus medullaris, cervical, thoracic and lumbar spinal cord, brain stem, hypothalamus and striatum. In each of these cases the isolated CNS stem cell exhibits self-maintenance and generates a large number of progenitor cells which differentiate into neurons, astrocytes and oligodendrocytes. Thus, multipotent stem cells are present in multiple regions of the adult mammalian CNS. CNS stem cells are isolated from dysfunctional CNS tissue, for exampletissue afflicted with Alzheimer's Disease, Parkinson's Disease, or Down's Syndrome.

The precursor cells described above can be used in methods of determining the effect of biological agents on neural cells. The term "biological agent" refers to any agent, such as a virus, protein, peptide, amino acid, lipid, carbohydrate, nucleic acid, nucleotide, drug, pro-drug or other substance that may have an effect on neural cells whether such effect is harmful, beneficial, or otherwise. Biological agents that are beneficial to neural cells are referred to herein as "neurological agents", a term which encompasses any biologically or pharmaceutically active substance that may prove potentially useful for the proliferation, survival, differentiation and/or functioning of CNS cells or treatment of neurological disease or disorder. For example, the term may encompass certain neurotransmitters, neurotransmitter receptors, growth factors, growth factor receptors, and the like, as well as enzymes used in the synthesis of these agents.

To determine the effect of a potential biological agent on neural cells, a culture of precursor cells derived from multipotent stem cells obtained from a host afflicted with a CNS disease or disorder can be used, or a culture obtained from normal tissue can be used. The choice of culture will depend upon the particular agent being tested and the effects one wishes to achieve. Once the cells are obtained from the desired donor tissue, they are proliferated in vitro in the presence of a growth factor.

The effects of biological agents on the proliferation and survival of stem cells and progenitor cells are determined using cells grown according to Example 1. For example, using these methods, it is possible to screen for biological agents that increase the proliferative ability of progenitor cells which would be useful for generating large numbers of cells for transplantation purposes. It is also possible to screen for biological agents which inhibit precursor cell proliferation. In these studies precursor cells are plated in the presence of the biological factor(s) of interest and assayed for the degree of proliferation which occurs (Example 4). The effects of a biological agent or combination of biological agents on the differentiation and survival of progenitor cells and their progeny can be determined (Example 3). It is possible to screen neural cells which have already been induced to differentiate prior to the screening. It is also possible to determine the effects of the biological agents on the differentiation process by applying them to precursor cells prior to differentiation. Generally, the biological agent will be solubilized and added to the culture medium at varying concentrations to determine the effect of the agent at each dose. The culture medium may be replenished with the biological agent every couple of days in amounts so as to keep the concentration of the agent somewhat constant.

Using these screening methods, it is possible to screen for potential drug side-effects on pre- and post-natal CNS cells by testing for the effects of the biological agents on stem cell and progenitor cell proliferation and on progenitor cell differentiation or the survival and function of differentiated CNS cells. The proliferated precursor cells are typically plated at a density of about 5-10 x 10⁶ cells/ml. If it is desired to test the effect of the biological agent on a particular differentiated cell type or a given make-up of cells, the ratio of neurons to glial cells obtained after differentiation can be manipulated by separating the different types of cells. For example, the 04 antibody (available from Boerhinger Mannheim) binds to oligodendrocytes and their precursors. Using a panning procedure, oligodendrocytes are separated out. Astrocytes can be panned out after a binding procedure using the RAN 2 antibody (available from ATCC). Tetanus toxin (available from Boerhinger Mannheim) can be used to select out neurons. By varying the trophic factors added to the culture medium used during differentiation it is possible to intentionally alter the phenotype ratios. Such trophic factors include EGF, FGF, BDNF, CNTF, TGFα, GDNF, and the like. For example, FGF increases the ratio of neurons, and CNTF increases the ratio of oligodendrocytes. Growing the cultures on beds of glial cells obtained from different CNS regions will also affect the course of differentiation as described above. Cultures can also be obtained that are enriched in dopaminergic neurons. These cultures can be used to test biological agents that influence dopaminergic cell function and survival. Culture conditions can also be varied to increase the numbers of cholinergic neurons. The differentiated cultures remain viable (with phenotype intact) for at least a month.

Cultures obtained from abnormal CNS tissue from patients afflicted with Alzheimer's disease, Parkinson's disease, Down's Syndrome, and the like, can be used to test for the effect of biological agents on abnormal tissue. For example, cultures obtained from a patient afflicted with Parkinson's disease could be used to test for the induction of dopaminergic cells. Cultures obtained from Alzheimer's or Down's Syndrome patients can be used to measure the effects of biological agents on the level of amyloid precursor protein (APP), which is abnormally high in these patients. Additionally, tissue obtained from a patient with Alzheimer's disease, a cholinergic related disorder, can be used to test the effects of biological agents on the induction of cholinergic neurons.

The effects of the biological agents are monitored at time intervals and are assessed on the basis of significant difference relative to control cultures with respect to criteria such as the ratios of expressed phenotypes (neurons: glial cells, or neurotransmitters or other markers), cell viability, proliferation, alterations in gene expression, and/or extent of apoptosis. Physical characteristics of the cells can be analyzed by observing cell and neurite morphology and growth with microscopy. The induction of expression of new or increased levels of proteins such as enzymes, receptors and other cell surface molecules, or of neurotransmitters, amino acids, neuropeptides and biogenic amines can be analyzed with any technique known in the art which can identify the alteration of the level of such molecules. These techniques include immunohistochemistry using antibodies against such molecules, or biochemical analysis. Such biochemical analysis includes protein assays, enzymatic assays, receptor binding assays, enzyme-linked immunosorbant assays (ELISA), electrophoretic analysis, analysis with high performance liquid chromatography (HPLC), Western blots, and radioimmune assays (RIA). Nucleic acid analysis such as Northern blots and polymerase chain reaction (PCR) can be used to examine the levels of mRNA coding for these molecules, or for enzymes which synthesize these molecules. Genomic DNA can be quantified using standard procedures and analyzed for extent of DNA laddering (i.e. enzyme-specific breakdown of DNA), which is indicative of apoptosis.

The factors involved in the proliferation of stem cells and the proliferation, differentiation and survival of stem cell progeny, and/or their responses to biological agents can be isolated by constructing cDNA libraries from stem cells or stem cell progeny at different stages of their development using techniques known in the art. The libraries from cells at one developmental stage are compared with those of cells at different stages of development to determine the sequence of gene expression during development and to reveal the effects of various biological agents or to reveal new biological agents that alter gene expression in CNS cells. When the libraries are prepared from dysfunctional tissue, genetic factors may be identified that play a role in the cause of dysfunction by comparing the libraries from the dysfunctional tissue with those from normal tissue. This information can be used in the design of therapies to treat the disorders. Additionally, probes can be identified for use in the diagnosis of various genetic disorders or for use in identifying neural cells at a particular stage in development.

Electrophysiological analysis can be used to determine the effects of biological agents on neuronal characteristics such as resting membrane potential, evoked potentials, direction and ionic nature of current flow and the dynamics of ion channels. These measurements can be made using any technique known in the art, including extracellular single unit voltage recording, intracellular voltage recording, voltage clamping and patch clamping. Voltage sensitive dyes and ion sensitive electrodes may also be used.
In order that the invention described herein may be more fully understood, the following examples are set forth. It should be understood that these examples are for illustrative purposes only and are not to be construed as limiting the scope of the invention in any manner.

### Example 1

### Propagation of precursor cells

Embryonic day 14 (E14) CD₁ albino mice (Charles River) were decapitated and the brain and striata removed using sterile procedure. The tissue was mechanically dissociated with a fire-polished Pasteur pipette into serum-free medium composed of a 1:1 mixture of Dulbecco's modified Eagle's medium (DMEM) and F-12 nutrient mixture (Gibco). The cells were centrifuged at 800 r.p.m. for 5 minutes, the supernatant aspirated, and the cells resuspended in DMEM/F-12 medium for counting.

The cells were suspended in a serum-free medium, hereinafter referred to as "complete medium", composed of DMEM/F-12 (1:1) which included glucose (0.6%), glutamine (2 mM), sodium bicarbonate (3 mM), HEPES (4-[2-hydroxyethyl]-1-piperazineethanesulfonic acid) buffer (5 mM) and a defined hormone mix and salt mixture (to replace serum) that included insulin (25 *µ*g/ml), transferrin (100 *µ*g/ml), progesterone (20 nM), putrescine (60 *µ*M), and selenium chloride (30 nM) (all from Sigma except glutamine [Gibco]). In addition, the medium contained 16-20 ng/ml EGF (purified from mouse submaxillary, Collaborative Research) or TGFα (human recombinant, Gibco). The cells were plated at 0.2 x 10⁶ cells/ml into 75 cm² tissue culture flasks (Corning) with no substrate pre-treatment and housed in an incubator at 37°C, 100% humidity, 95% air/5% CO₂.

When the cells were proliferated, within the first 48 hours and by 3-4 days in vitro (DIV), they formed small clusters, known as neurospheres, that lifted off the substrate between 4-6 DIV (Fig. 1). Neurospheres contain undifferentiated precursor cells, i.e. stem cells and progenitor cells.

After 7 DIV, the neurospheres were removed, centrifuged at 400 r.p.m. for 2-5 minutes, and the pellet was mechanically dissociated into individual cells with a fire-polished glass Pasteur pipet in 2 mls of complete medium. 1 x 10⁶ cells were replated into a 75 cm² tissue culture flask with 20 mls of the EGF-containing complete medium. The proliferation of the stem cells and formation of new neurospheres was reinitiated. This procedure can be repeated every 6-8 days.

### Example 2

### Differentiation of neurospheres

Neurospheres were differentiated using the following paradigms. Using any of thefollowing paradigms will produce neurons, astrocytes and oligodendrocytes. However, adding certain growth factors or combinations of growth factors can alter the phenotype ratios obtained after differentiation. Additionally, the use of glial feeder beds can influence the phenotype ratios obtained. The neurospheres used for each of the following paradigms were generated as outlined in Example 1. All the neurospheres used were passed at least once prior to their differentiation.

### Paradigm 1 -- Rapid differentiation of neurospheres

Six to 8 days after the first passage, the neurospheres were removed and centrifuged at 400 r.p.m. The EGF-containing supernatant was removed and the pellet suspended in EGF-free complete medium containing 1 % fetal bovine serum (FBS).

Neurospheres (approximately 0.5-1.0 x 10⁶ cells/well) were plated on poly-L-ornithine-coated (15 *µ*g/ml) glass coverslips in 24 well Nuclon (1.0 ml/well) culture dishes. After 24 hours in culture, the coverslips were transferred to 12 well (Costar) culture dishes containing complete medium containing 0.5% FBS. The medium was changed every 4-7 days. This differentiation procedure is referred to as the "Rapid Differentiation Paradigm" or RDP.

### Paradigm 2 -- Differentiation of dissociated neurospheres

Six to 8 days after the first passage, the neurospheres were removed and centrifuged at 400 r.p.m. The EGF-containing media was removed and the pellet was suspended in EGF-free complete medium containing 1% FBS. The neurospheres were mechanically dissociated into single cells with a fire-polished Pasteur pipette and centrifuged at 800 r.p.m. for 5 minutes. Between 0.5 x 10⁶ and 1.0 x 10⁶ cells were plated on poly-L-ornithine-coated (15 *µ* g/ml) glass coverslips in 24 well Nuclon (1.0 ml/well) culture dishes. The EGF-free culture medium containing 1% FBS was changed every 4-7 days.

### Paradigm 3 -- Differentiation of single neurospheres

Neurospheres were washed free of EGF by serial transfers through changes of EGF-free medium. Individual neurospheres were plated onto poly-L-ornithine-coated (15 *µ*g/ml) glass coverslips in a 24-well plate. The culture medium used was complete medium with or without 1 % FBS. The medium was changed every 4-7 days. Triple label immunocytochemistry revealed that all three neural cell types, i.e. neurons, astrocytes and oligodendrocytes, are clonally derived from a single neurosphere (Fig. 2).

### Paradigm 4 -- Differentiation of single dissociated neurospheres

Neurospheres were washed free of EGF by serial transfers through changes of EGF-free medium. A single neurosphere was mechanically dissociated in a 0.5 ml Eppendorf centrifuge tube and all the cells were plated onto a poly-L-ornithine coated 35 mm culture dish. Complete medium was used with or without 1% FBS.

### Paradigm 5 -- Differentiation of neurospheres co-cultured with striatal astrocytes

Neurospheres, derived from striatal cells as described in Example 1 were labeled with 5-bromodeoxyuridine (BrdU) and washed free of EGF. An astrocyte feeder layer was generated from striatal tissue of postnatal mice (0-24 hours), and plated on poly-L-ornithine-coated glass coverslips in a 24-well culture dish. When the astrocytes were confluent, a dissociated or intact neurosphere was placed on each astrocyte bed. Complete medium was changed after the first 24 hours and then every forty-eight hours. When differentiated on an astrocyte feeder layer, in addition to GABAergic and Substance Pergic neurons, somatostatin, NPY, glutamate and methenkephalin-containing neurons were present (Fig. 3).

### Example 3

### Screening of Drugs or Other Biological Agents for Various Effects

### A. Effects of BDNF on Neuronal and Glial Cell Differentiation and Survival

Precursor cells were propagated as described in Example 1 and differentiated using Paradigm 3 described in Example 2. At the time of plating the EGF-generated cells, BDNF was added at a concentration of 10 ng/ml. At 3, 7, 14, and 21 days in vitro (DIV), cells were processed for indirect immunocytochemistry. BrdU labeling was used to monitor proliferation of the precursor cells. The effects of BDNF on neurons, oligodendrocytes and astrocytes were assayed by probing the cultures with antibodies that recognize antigens found on neurons (MAP-2, NSE, NF), oligodendrocytes (O4, GalC, MBP) or astrocytes (GFAP). Cell survival was determined by counting the number of immunoreactive cells at each time point and morphological observations were made. BDNF significantly increased the differentiation and survival of neurons over the number observed under control conditions (Fig. 4). Astrocyte and oligodendrocyte numbers were not significantly altered from control values.

### B. Effects of BDNF on the Differentiation of Neural Phenotypes

Cells treated with BDNF according to the methods described in Part A were probed with antibodies that recognize neural transmitters or enzymes involved in the synthesis of neural transmitters. These included tyrosine hydroxylase (TH), choline acetyltransferase (ChAT), substance P, GABA, somatostatin, and glutamate. In both control and BDNF-treated culture conditions, neurons tested positive for the presence of substance P and GABA. (FIG. 5). As well as an increase in numbers, neurons grown in BDNF showed a dramatic increase in neurite extension and branching when compared with control examples (Fig. 6).

### C. Identification of Growth-Factor Responsive Cells

Cells that are responsive to growth factor treatment were identified by differentiating the EGF-generated progeny as described in Example 2, paradigm 3 and at 1 DIV adding approximately 100 ng/ml of BDNF. At 1, 3, 6, 12 and 24 hours after the addition of BDNF the cells were fixed and processed for dual label immunocytochemistry. Antibodies that recognize neurons (MAP-2, NSE, NF), oligodendrocytes (O4, GalC, MBP) or astrocytes (GFAP) were used in combination with an antibody that recognizes c-fos and/or other immediate early genes. Exposure to BDNF results in a selective increase in the expression of c-fos in neuronal cells (Fig. 6).

### D. Effects of BDNF on the Expression of Markers and Regulatory Factors During Proliferation and Differentiation

Cells treated with BDNF according to the methods described in Part A are processed for analysis of the expression of FGF-R1, as described in Example 5 or other markers and regulatory factors, as described in Example 6.

### E. Effects of BDNF administration During Differentiation on the Electrophysiological Properties of Neurons

Neurons treated with BDNF during differentiation, according to the methods described in Part A, are processed for the determination of their electrophysiological properties, as described in Example 7.

### F. Effects of Chlorpromazine on the Proliferation, Differentiation, and Survival of Growth Factor Generated Stem Cell Progenv

Chlorpromazine, a drug widely used in the treatment of psychiatric illness, is used in concentrations ranging from 10 ng/ml to 1000 ng/ml in place of BDNF in Examples 3A to 3E above. The effects of the drug at various concentrations on stem cell proliferation and on stem cell progeny differentiation and survival is monitored. Alterations in gene expression and electrophysiological properties of differentiated neurons are determined.

### G. Effects of Deprenyl on the Differentiation, and Survival of Dopaminergic Cells

Primary cultures are prepared using the methods in Example 1. The cells are differentiated to increase the number of dopaminergic neurons. Single undissociated 6-day old primary generated neurospheres are plated onto poly-L-ornithine coated glass coverslips in complete medium with rat B-49 glial-cell line derived conditioned medium (75%) + 20 ng/ml FGF-2, and incubated at 37°C, 100% humidity, 95% air/5% CO₂. Deprenyl, a drug used in the treatment of Parkinson's disease, is added to the cultures in concentrations ranging from 1 ng/ml to 1000 ng/ml at the onset of differentiation and/or once differentiation has occurred. The number of surviving dopaminergic cells are counted at intervals and compared to control cultures. In addition, biochemical assays to measure neurotransmitter expression and nucleic acid analysis is undertaken.

### Example 4

### Stem Cell Proliferation Assay

Primary cells were obtained from E14 mice and prepared as outlined in Example 1. Either EGF, EGF and FGF or EGF and BMP-2 were added to complete medium at a concentration of 20 ng/ml of each growth factor, with the exception of BMP-2 which was added at a concentration of 10 ng/ml. Cells were diluted with one of the prepared growth factor-containing media to a concentration of 25,000 cells/ml. 200 *µ*l of the cell/medium combination were pipetted into each well of a 96-well place (Nuclon) with no substrate pretreatment. Cells were incubated under the same conditions as outlined in Example 1.

After 8-10 DIV the number of neurospheres was counted and the results tabulated. As indicated in Fig. 7, cells grown in a combination of EGF and FGF produced significantly more neurospheres than cells grown in the presence of EGF alone. The combination of EGF and BMP-2 inhibited neurosphere development.

### Example 5

### Comparison of Receptor and Growth Factor Expression in Undifferentiated vs. Differentiated Stem Cell-Derived Progeny by Reverse Transcription-Polymerase Chain Reaction (RT-PCR)

Neurospheres were generated as described in Example 1, and some were differentiated as per Paradigm 1, Example 2. RNA from either undifferentiated or differentiated neurospheres was isolated according to the guanidinium thiocyanate acid phenol procedure of Chomzynski and Sacchi -Anal. Biochem. 162: 156-159 1987)]. Complementary DNA (cDNA) was synthesized from total RNA using reverse transcriptase primed with oligo dT. Gene-specific primers were designed and synthesized and these primers were used in PCR to amplify cDNAs for different growth factors and growth factor receptors. Amplified material was run on agarose gels alongside molecular weight markers to ensure that PCR products were of the expected size, while the identity of PCR fragments was confirmed by restriction enzyme analysis and by sequencing [Arcellana-Panlilio, Methods Enzymol. 225: 303-328 (1993)]. Fig. 8 is a photograph of an ethidium-stained agarose gel visualized via UV transillumination showing the detection of three growth factor receptor transcripts, namely EGF-R, FGF-R, and LIF-R, in undifferentiated and differentiated stem cell-derived progeny. Table I lists the primer sets analyzed and the results of undifferentiated and differentiated cells.

**TABLE I**

| Primer Sets Analyzed | | |
|---|---|---|
| | Undifferentiated Cells | Differentiated Cells |
| Actin | + | + |
| NGF | + | nd |
| EGFr^{m} | + | + |
| bFGFr | + | + |
| LIFr^{m} | + | + |
| tyrosine hydroxylase | + | + |
| choline acetyltransferase^{m} | nd | + |
| cholecystokinin^{m} | nd | - |
| enkephalin^{m} | nd | + |
| tyrosine kinase-rA | + | + |
| tyrosine kinase-rB | + | +++++ |
| tyrosine kinase-rC | + | + |
| r = receptor | | |
| m = derived from mouse | | |
| nd = no data available | | |

### Example 6

### Isolation of Novel Markers and Regulatory Factors Involved in Neural Stem Cell Proliferation and Differentiation

Neurospheres are generated as described in Example 1 using CNS tissue from CD₁ albino mice (Charles River). Some of these neurospheres are allowed to differentiate according to the rapid differentiation paradigm of Example 2 producing cultures enriched in neurons, astrocytes, and oligodendrocytes. Total RNA is extracted from the undifferentiated neurospheres as well as the differentiated cell cultures using the guanidinium thiocyanate acid phenol method referred to in Example 5. Messenger RNA (mRNA) is isolated by exploiting the affinity of its poly A tract to stretches of either U's or T's. Reverse transcription of the mRNA produced cDNA, is then used to make primary libraries in either plasmid [Rothstein et al., Methods in Enzymology 225:587-610 (1993)] or lambda phage vectors. To isolate cDNAs that are specific to either undifferentiated or differentiated stem cell derived progeny, cDNA from one is hybridized to RNA from the other, and vice versa. The unhybridized, and thus culture type-specific, cDNAs in each case are then used to construct subtracted libraries [Lopez-Fernandez and del Mazo, Biotechniques 15(4):654-658 (1993)], or used to screen the primary libraries.

Stem cell-derived undifferentiated cell specific and differentiated cell specific cDNA libraries provide a source of clones for novel markers and regulatory factors involved in CNS stem cell proliferation and differentiation. Specific cDNAs are studied by sequencing analysis to detect specific sequence motifs as clues to identity or function, and database searching for homologies to known transcripts. Using cDNAs in a hybridization to various RNA samples electrophoresed on an agarose-formaldehyde gel and transferred to a nylon membrane, allows the estimation of size, relative abundance, and specificity of transcripts. All or portions of cDNA sequences are used to screen other libraries in order to obtain either complete mRNA sequences or genomic sequence information. Antibodies directed against fusion proteins generated from specific cDNAs are used to detect proteins specific to a particular cell population, either by immunocytochemistry or by Western Blot analysis. Specific gene sequences are used to isolate proteins that interact with putative regulatory elements that control gene expression. These regulatory elements are then used to drive the expression of an exogenous gene, such as beta-galactosidase.

### Example 7

### Electrophysiological Analysis of Neurons Generated From Growth Factor-Responsive Stem Cells and Exposed to a Biological Agent

Neurospheres were generated as described in Example 1. Neurospheres were dissociated using the technique described in paradigm 2, Example 2. The clonally derived cells were plated at low density and differentiated in the presence of bFGF. The electrophysiological properties of cells with the morphological appearance of neurons were determined as described as described by Vescovi et al. [Neuron, 11: 951-966 (1993)]. Under whole cell current clamp, the mean resting potential and input resistance were -62 ± 9mV and 372 ± MΩ. Rectangular suprathreshold current steps, (^{~}100 pA) elicited regenerative potential responses in which the amplitude and time course were stimulus dependent (Fig. 9). After the completion of electrophysiological experiments, the cell morphology was visualized by intracellular excitation of 5-carboxyfluorescein.

### Example 8

### Screening for the Effects of Drugs or Other Biological Agents on Growth Factor-Responsive Stem Cell Progeny Generated From Tissue Obtained From a Patient with a Neurological Disorder

The effects of BDNF on the EGF-responsive stem cell progeny generated from CNS tissue obtained at biopsy from a patient with Huntington's disease is determined using the methods outlined in Example 3, A to E. BDNF is a potent differentiation factor for GABAergic neurons and promotes extensive neuronal outgrowth (Fig. 5B). Huntington's Disease is characterized by the loss of GABAergic neurons (amongst others) from the striatum.

### Example 9

### Regulation of Amyloid Precursor Protein (APP) by Growth Factors

CNS tissue is obtained from a Down's Syndrome fetus and neurospheres are generated using the methods of Example 1 and passaged to obtain the required number of cells. The cells are differentiated using any of the paradigms described in Example 2. At the time of plating, CNTF, BMP-2, activin, FGF-2, or retinoic acid is added to the culture medium in the experimental wells at a concentration of 10 ng/ml and added every other day at a concentration of 2 ng/ml. After 3, 7 and 14 DIV, the levels of APP mRNA and protein are determined. For Northern Blot analysis, RNA is extracted using the guanidinium isothiocyanate/cesium chloride method [Goodison et al., J. Neuropathol. Exp. Neurol. 52(3): 192-198 (1993)]. Northern blots are run and probed using a human cDNA encoding the protease inhibitor domain of APP_{KPI} or a 30 base pair oligonucleotide probe specific for APP₆₉₅. For Western Blot analysis, cells are homogenized in Laemmli buffer, boiled and subjected to an SDS-PAGE gel. The gel is immunoblotted and probed with anti-APP diluted 1:1000. Levels of APP mRNA and protein expression are compared to control cultures.

### Example 10

### Analysis of Apoptotic Events Using Proliferated Neural Stem Cell Progeny

### A. Analysis of Spontaneous Apoptosis

Proliferating mouse neurospheres were prepared using the methods of Example 1 and harvested after 3, 5, 7, 9, 12 and 15 days of culture. Mouse neurospheres were differentiated using the method of Example 2, paradigm 1 and harvested after 1, 4, 7, 10, 13 and 16 days of culture. Cells were lysed in 1 ml DNAzol reagent (Gibco/BRL) and genomic DNA was pooled from solution following precipitation with 500 *µ* l ethanol. Genomic DNA was quantified by optical density at 260 nm. The extent of DNA laddering indicative of apoptosis was detected by dissolving 250 ng of DNA in 50 *µ*l 100 mM potassium cacodylate (pH 7.2), 2 mM CoCl₂, 0.2 mM DTT, 50 *µ* Ci [α³²P]dATP and 25 units terminal deoxynucleotidyl transferase. The reaction was incubated for 60 minutes at 37°C. The radioactive products were analyzed by 2% gel electrophoresis and autoradiography. Morphological and biochemical analysis of proliferating and differentiated neurosphere cultures indicate that the number of cells actively engaged in spontaneous apoptosis range from less than 20% to greater than 50% with increasing days of culture.

### B. RT-PCR Analysis of Potential Regulators of Apoptosis in Neuronal Stem Cell Cultures

The activity of known putative apoptosis regulatory molecules were ascertained by RT-PCR analysis. Proliferating and differentiated progeny of murine neuronal stem cell cultures were prepared using the methods of Examples 1 and 2. Reverse transcription-PCR analysis of known putative apoptosis regulatory mRNA transcripts was undertaken. Cells were lysed in 1 ml RNAzol reagent (Gibco/BRL), organic and aqueous phases were separated by addition of 0.2 volumes chloroform and total RNA was isolated from the aqueous phase by precipitation with addition of an equal volume of isopropanol. RNA was quantified by optical density at 260 and 280 nm. Polymerase Chain Reaction analysis of 0.5 *µ*l reverse transcription product was undertaken in 25 *µ*L 20 mM Tris-HCI (pH 8.0), 50 mM KCI, 0.2 mM dNTPs, 1.5 mM MgCl₂, 0.5 *µ*M primers and 1.25 units taq polymerase. Typical cycling parameters were 94°C for 30 seconds, 60°C for 30 seconds and 72°C for 1 minute repeated for 30 cycles. Over thirty potential regulators of neuronal apoptosis were analyzed including members of growth factors, growth factor receptors, transcription factors, the Bcl-2 protein family members and the interleukin converting enzyme family of proteases. Differentially expressed members of each protein family was detected.

### C. Detection and Cloning of Unknown Potential Regulators of Neuronal Apoptosis

The activity of unknown transcripts potentially regulating neuronal apoptosis was ascertained utilizing mRNA fingerprinting analysis. Proliferating and differentiated progeny of murine neuronal stem cell cultures were prepared and harvested as described above and mRNA fingerprinting analysis of unknown putative apoptosis regulatory molecules was undertaken. Cells were lysed in 1 ml RNAzol reagent (Gibco/BRL), organic and aqueous phases were separated by addition of 0.2 volumes chloroform and total RNA was isolated from the aqueous phase by precipitation with addition of an equal volume of isopropanol. RNA was quantified by optical density at 260 and 280 nm. Reverse transcription and Polymerase Chain Reaction analysis was undertaken as described above. Radioactive products were separated on 8% acrylamide sequencing gels and analyzed by autoradiography. Differentially expressed bands were cut out of the gel, re-amplified and sequenced.

### D. Establishment of Genetically Modified Stem Cells For High Throughput Assay of Anti-Apoptosis Compounds

Human and murine neuronal stem cells are genetically modified in order to provide a high-throughput assay system for potentially therapeutic anti-apoptosis compounds. Using a number of direct transfection techniques, DNA constructs containing cytoplasmic (green fluorescent protein (GFP) or secreted (secreted alkaline phosphatase (SEAP)) marker proteins driven by apoptosis regulatory molecule promoters (including Bcl-2, ICE and Nur-77) are stably transformed into murine and human neuronal stem cells. For transformation utilizing Lipofectamine (BRL), cells are seeded at 2-3 x 10⁶ cells per 35-mm culture plate and incubated with 200 µl DNA-liposome complexes (3 µg DNA, 20 µl lipofectamin (BRL) in 200 µl media) for 12 hours at 37°C. The effect of a variety of compounds on neuronal apoptosis is ascertained by the effect that the application of the compound has on the expression of the marker genes under the control of known apoptosis regulatory gene promoters by fluorescence (GFP) or secreted alkaline phosphatase activity (SEAP).

## Claims

1. A method for determining the potential therapeutic effect of at least one biological agent on a neurological disease or disorder comprising:
(a) obtaining cells derived from dissociated dysfunctional mammalian neural CNS tissue afflicted with said neurological disease or disorder, said tissue containing at least one multipotent neural stem cell,
(b) culturing said multipotent neural stem cell in a culture medium containing at least one growth factor that induces multipotent neural stem cell proliferation to obtain a culture of proliferated neural cells,
(c) contacting said culture of proliferated neural cells with said biological agent, and
(d) determining the effects of said biological agent on said proliferated neural cells, wherein the presence of an effect indicates the potential therapeutic effect of said biological agent.

2. The method of claim 1, wherein said growth factor is selected from the group consisting of EGF, bFGF, or a combination of EGF and bFGF.

3. The method of claim 1 or 2, wherein said culture medium is serum-free.

4. The method of any one of claims 1 to 3, wherein said mammalian neural CNS tissue is obtained from a postnatal mammal.

5. The method of claim 4, wherein said mammal is human.

6. The method of any one of claims 1 to 5, wherein said neurological disease or disorder is selected from the group consisting of Alzheimer's Disease, Parkinson's Disease, Huntington's Disease or Down's Syndrome.

7. The method of any one of claims 1 to 6, wherein said effects of step (d) are determined by comparing a gene library of the proliferated neural cells of step (c) which have been contacted with said biological agent with a gene library of the proliferated neural cells of step (b) which have not been in contact with said biological agent.

8. The method of any one of claims 1 to 7, wherein prior to step (c) the proliferated neural cells are induced to differentiate in a second culture medium.

9. The method of any one of claims 1 to 7, wherein in step (c) the proliferated neural cells are induced to differentiate in the presence of said biological agent.

10. The method of claim 8 or 9, wherein said biological agent is a trophic factor that alters the phenotype of said differentiated cells.

11. The method of claim 8, wherein said second culture medium comprises a glial feeder-cell layer.

12. The method of claim 9, wherein said proliferated neural cells are induced to differentiate in the presence of a glial feeder-cell layer.

## Patentansprüche

1. Verfahren zur Bestimmung der potentiellen therapeutischen Wirkung von mindestens einem biologischen Agens gegen eine neurologische Krankheit oder Störung, umfassend:
(a) Gewinnen von Zellen aus dissoziiertem geschädigtem neuronalen Gewebe aus dem ZNS von Säugern, die von der neuronalen Krankheit oder Störung befallen sind, wobei dieses Gewebe mindestens eine multipotente neuronale Stammzelle enthält;
(b) Züchten der multipotenten neuronalen Stammzelle in Kulturmedium, welches mindestens einen Wachstumsfaktor enthält, der die Proliferation multipotenter neuronaler Stammzellen induziert, so dass man eine Kultur proliferierter neuronaler Zellen erhält;
(c) Inkontaktbringen der Kultur proliferierter neuronaler Zellen mit dem biologischen Agens; und
( d) Bestimmen der Wirkung des biologischen Agens auf die proliferierten neuronalen Zellen, wobei die Beobachtung eines Effekts die potentielle therapeutische Wirkung des biologischen Agens anzeigt.

2. Verfahren nach Anspruch 1, wobei der Wachstumsfaktor aus der Gruppe ausgewählt wird, bestehend aus EGF, bFGF oder einer Kombination aus EGF und bFGF.

3. Verfahren nach Anspruch 1 oder 2, wobei das Kulturmedium Serum-frei ist.

4. Verfahren nach einem der Ansprüche 1 bis 3, wobei das neuronale Gewebe aus dem ZNS von Säugern aus einem solchen Säuger nach der Geburt gewonnen wird.

5. Verfahren nach Anspruch 4, wobei der Säuger ein Mensch ist.

6. Verfahren nach einem der Ansprüche 1 bis 5, wobei die neuronale Krankheit oder Störung ausgewählt ist aus der Gruppe bestehend aus Alzheimer-Krankheit, Parkinson-Krankheit, der Huntington-Krankheit oder dem Down-Syndrom.

7. Verfahren nach einem der Ansprüche 1 bis 6, wobei die Wirkungen von Schritt (d) bestimmt werden durch Vergleich einer Genbibliothek der proliferierten neuronalen Zellen von Schritt (c), die mit dem biologischen Agens in Kontakt gebracht wurden, mit einer Genbibliothek von proliferierten neuronalen Zellen von Schritt (b), welche nicht mit dem biologischen Agens in Kontakt gebracht wurden.

8. Verfahren nach einem der Ansprüche 1 bis 7, wobei vor Schritt (c) eine Differenzierung der proliferierten neuronalen Zellen in einem zweiten Kulturmedium induziert wird.

9. Verfahren nach einem der Ansprüche 1 bis 7, wobei in Schritt (c) eine Differenzierung der proliferierten neuronalen Zellen in Anwesenheit des biologischen Agens induziert wird.

10. Verfahren nach Anspruch 8 oder 9, wobei das biologische Agens ein trophischer Faktor ist, der den Phänotyp der differenzierten Zellen verändert.

11. Verfahren nach Anspruch 8, wobei das zweite Kulturmedium eine Schicht von Glia-Feederzellen beinhaltet.

12. Verfahren nach Anspruch 9, wobei die Differenzierung der proliferierten neuronalen Zellen in Anwesenheit einer Schicht von Glia-Feederzellen induziert wird.

## Revendications

1. Procédé pour déterminer l'effet thérapeutique potentiel d'au moins un agent biologique sur un trouble ou une affection neurologique, comprenant les opérations consistant à :
(a) obtenir des cellules dérivées de tissu du SNC neural dysfonctionnel dissocié de mammifère, atteint dudit trouble ou affection neurologique, ledit tissus contenant au moins une cellule souche neurale multipotente,
(b) mettre en culture ladite cellule souche neurale dans un milieu de culture contenant au moins un facteur de croissance qui induit une prolifération de cellules souches neurales multipotentes pour obtenir une culture de cellules neurales proliférées,
(c) mettre en contact ladite culture de cellules neurales proliférées avec ledit agent biologique, et
(d) déterminer les effets dudit agent biologique sur lesdites cellules neurales proliférées, la présence d'un effet indiquant l'effet thérapeutique potentiel dudit agent biologique.

2. Procédé selon la revendication 1, dans lequel le facteur de croissance est choisi dans le groupe consistant en EGF, bFGF, ou une combinaison de EGF et bFGF.

3. Procédé selon la revendication 1 ou 2, dans lequel ledit milieu de culture est exempt de sérum.

4. Procédé selon l'une quelconque des revendications 1 à 3, dans lequel ledit tissu du SNC neural de mammifère est obtenu à partir d'un mammifère postnatal.

5. Procédé selon la revendication 4, dans lequel ledit mammifère est un humain.

6. Procédé selon l'une des revendications 1 à 5, dans lequel ledit trouble ou ladite affection neurologique est choisi dans le groupe consistant en la maladie d'Alzheimer, la maladie de Parkinson, la maladie de Huntincton ou le syndrome de Down.

7. Procédé selon l'une quelconque des revendications 1 à 6, dans lequel lesdits effets de l'étape (d) sont déterminés en comparant une banque génomique des cellules neurales proliférées de l'étape (c) qui ont été mises en contact avec ledit agent biologique avec une banque génomique des cellules neurales proliférées de l'étape (b) qui n'ont pas été mises en contact avec ledit agent biologique.

8. Procédé selon l'une quelconque des revendications 1 à 7, dans lequel avant l'étape (c), les cellules neurales proliférées sont induites à se différencier dans un second milieu de culture.

9. Procédé selon l'une quelconque des revendications 1 à 7, dans lequel à l'étape (c), les cellules neurales proliférées sont induites à se différencier en présence dudit agent biologique.

10. Procédé selon la revendication 8 ou 9, dans lequel ledit agent biologique est un facteur trophique qui modifie le phénotype desdites cellules différenciées.

11. Procédé selon la revendication 8, dans lequel ledit second milieu de culture comprend une couche de cellules-alimentateur glial.

12. Procédé selon la revendication 9, dans lequel lesdites cellules neurales proliférées sont induites à se différencier en présence d'une couche de cellules-alimentateur glial.
